# EUROPEAN PATENT APPLICATION

(11) **EP 1 604 998 A2**
(43) Date of publication of application: **14.12.2005**
(21) Application number: 05018455.5
(22) Date of filing: 20.06.2001
(51) Int. Cl.: C08B 30/04, C12N 9/50, C12S 3/12

(54) **Starch washing process**

(30) Priority: 30.06.2000 DK 200001021; 10.07.2000 US 217130 P
(62) Divisional of application: 01943182.4
(71) Applicant: Novozymes A/S, 2880 Bagsvaerd (DK)
(72) Inventor: Olsen, Hans Sejr, 2840 Holte (DK); Nielsen, Bjarne Rönfeldt, 2830 Virum (DK)

(57) **Abstract**

The present invention relates to an improved process of washing a starch slurry obtained from the starch gluten separation step of a milling process, comprising washing the starch slurry with an aqueous solution comprising an effective amount of acidic protease.

## Description

### FIELD OF THE INVENTION

The present invention relates to an improved starch washing process constituting the final step in milling processes with the object of processing corn kernels and other crop kernels into high quality starch suitable for the conversion of starch into mono- di-, oligosaccharides, ethanol, sweeteners etc.

Further the invention also relates to an enzyme composition comprising a protein degrading activities for use in a starch gluten separation process.

### BACKGROUND OF THE INVENTION

Before starch - being an important constituent in the kernels of most crops, such as corn, wheat, rice, sorghum bean, barley or fruit hulls - can be used for conversion into saccharides, such as dextrose, fructose; alcohols, such as ethanol; and sweeteners, the starch must be made available and treated in an manner to provide a high purity starch. If starch contains more than 0.5% impurities, including proteins, it is not suitable as starting material for starch conversion processes. To provide such pure starch product staring out from the kernels of crops the kernels are often milled, as will be described further below.

### The Composition of Corn Kernels

Corn kernels, such as the yellow dent corn kernel, have an outer covering referred to as the "Pericarp" that protects the germ in the kernels. It resists water and water vapour and is undesirable to insects and microorganisms.

The only area of the kernels not covered by the "Pericarp" is the "Tip Cap", which is the attachment point of the kernel to the cob.

The "Germ" is the only living part of the corn kernel. It contains the essential genetic information, enzymes, vitamins, and minerals for the kernel to grow into a corn plant. About 25 percent of the germ is corn oil. The endosperm covered surrounded by the germ comprises about 82 percent of the kernel dry weight and is the source of energy (starch) and protein for the germinating seed. There are two types of endosperm, soft and hard. In the hard endosperm, starch is packed tightly together. In the soft endosperm, the starch is loose.

### Wet milling

Wet milling is often used for separating corn kernels into its four basic components: starch, germ, fiber and protein.

Typically-wet milling processes comprise four basic steps. First the kernels are steeped for 30 to 48 hours to begin breaking the starch and protein bonds. The next step in the process involves a coarse grind to separate the germ from the rest of the kernel. The remaining slurry consisting of fiber, starch and protein is finely ground and screened to separate the fiber from the starch and protein. The starch is separated from the remaining slurry in hydrocyclones. The starch then can be converted to syrup or alcohol.

### Dry Milling

In dry milling processes crop kernels, in particular corn kernels, are grinded in substantially dry state, without pre-soaking the kernels to separate the kernels into its major constituents: starch, germ, fiber and protein.

Today enzymes are not commonly for wet or dry milling of crop kernels. However, the use of enzymes has been suggested for the steeping step of wet milling processes.

The commercial enzyme product Steepzyme® (available from Novozymes A/S) have been shown suitable for the first step in wet milling processes, i.e., the steeping step where corn kernels are soaked in water.

Accordingly, the object of the invention is to provide an improved starch washing process.

### BRIEF DESCRIPTION OF THE DRAWINGS

Figure 1 shows a corn wet milling process,
Figure 2 shows the processes used in a corn steeping plant in a schematic form.

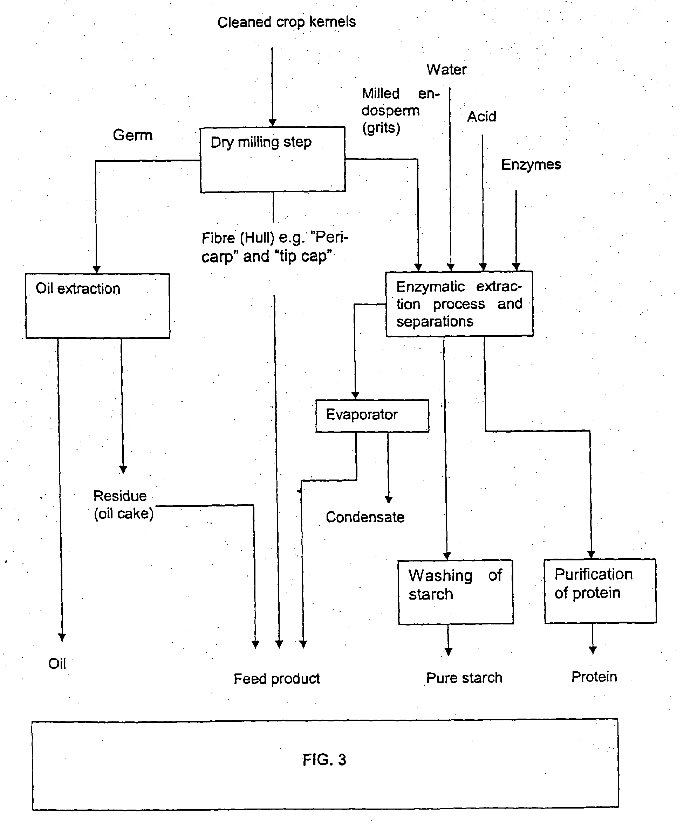

Figure 3 shows a dry milling process including starch and protein recovery. In the case of corn the feed product is similar to corn gluten and the protein is similar to corn gluten meal.

### DETAILED DESCRIPTION OF THE INVENTION

The object of the present invention is to provide an improved starch washing process.

When using the term "kernels" it is intended to include kernels from corn (maize), rice, barley, sorghum bean, or fruit hulls, or wheat.

In the context of the present invention, the term "enriched" is intended to indicate that the enzyme activity in question of the enzyme preparation has been increased, e.g., with an enrichment factor of at least 1.1, conveniently due to addition of a recombinant mono-component enzyme.

### The Milling Process

The kernels are milled in order to open up the structure and to allow further processing. Two processes are used: wet or dry milling. In dry milling processes the whole kernels are milled and used in the remaining part of the process. Wet milling gives a very good separation of germ and meal (starch granules and protein).

### Dry Milling

Dry milling processes are well known in the art. The term "dry milling" is in the context of the invention meant to include all such processes where the kernels are grinded in dry state.

Dry milling may for instance be carried out as follows: Dry kernels are first cleaned to remove chaff and other external vegetable matter. The hulls of the cleaned dry kernels are intentionally broken to facilitate subsequent milling, and passed through an impact-degerminating mill to loosen up the kernels germ. The discharge from the degerminating mill, comprising germ, fibre (hull) and endosperm (which is the raw material for the starch recovery process), is sifted into fractions according to particle size. The sifted fractions are subjected to suction using air aspirators, which separates the hull fiber. The dehulled discharge from the air aspirators, comprising germ and endosperm, is passed over vibrating gravity tables to separate the germ from the endosperm. The germ is collected from the gravity tables and, if desired, sent to oil expelling station.

### Wet milling

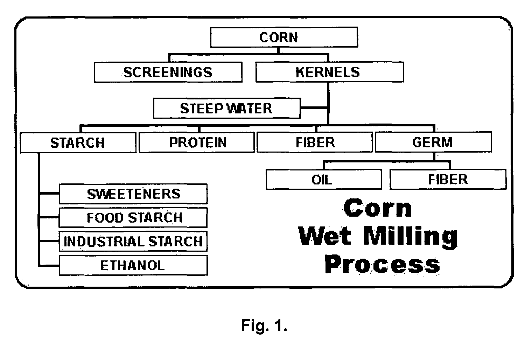

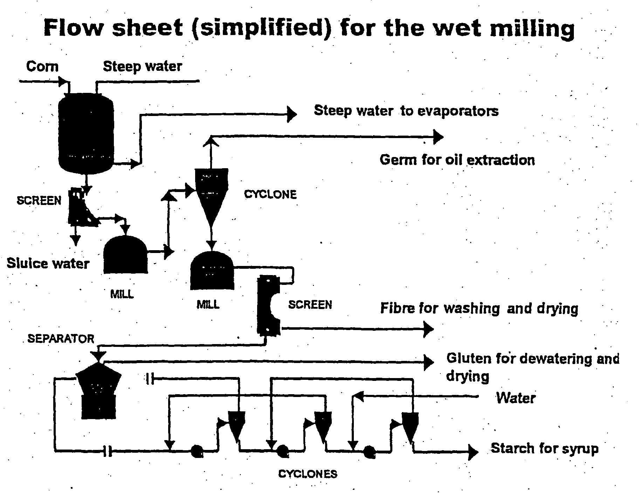

Degradation of the kernels of corn (see also Fig. 1 and Fig. 2) and other crop kernels into starch suitable for conversion of starch into mono-, di-, oligo saccharides, ethanol, sweeteners etc. consists of four main steps:
1. Steeping and germ separation,
2. Fiber washing and drying,
3. Starch gluten separation,
4. Starch washing.

### 1. Steeping and germ separation

Corn kernels are softened by soaking in water for between 30 and 48 hours at a temperature around 50□C. During steeping, the kernels absorb water, increasing their moisture levels from 15% to 45% and more than doubling in size. The addition of 0.1% sulfur dioxide (SO₂) and/or NaHSO₃ to the water prevents excessive bacteria growth in the warm environment. As the corn swells and softens, the mild acidity of the steepwater begins to loosen the gluten bonds within the corn and release the starch. After the corn kernels are steeped they are cracked open to release the germ. The germ contains the valuable corn oil. The germ is separated from the heavier density mixture of starch, hulls and fiber essentially by "floating" the germ segment free of the other substances under closely controlled conditions. This method serves to eliminate any adverse effect of traces of corn oil in later processing steps.

### 2. Fiber washing and drying

To get maximum starch recovery, while keeping any fiber in the final product to an absolute minimum, it is necessary to wash the free starch from the fiber during processing. The fiber is collected, slurried and screened to reclaim any residual starch or protein.

### 3. Starch separation

The starch-gluten suspension from the fiber-washing step, called mill starch, is separated into starch and gluten. Gluten has a low density compared to starch. By passing mill starch through a centrifuge, the gluten is readily spun out.

### 4. Starch washing

The starch slurry from the starch separation step contains some insoluble protein and much of solubles. They have to be removed before a top quality starch (high purity starch) can be made. The starch, with just one or two percent protein remaining, is diluted, washed 8 to 14 times, re-diluted and washed again in hydroclones to remove the last trace of protein and produce high quality starch, typically more than 99.5 percent pure.

### Process Of The Invention

The inventors of the-present invention have surprisingly found that acidic protease activity may advantageously be used for efficiently removing insoluble protein and solubles from the starch slurry obtained from the starch separation step. In the first aspect the invention relates to a process of washing the starch slurry obtained from the starch gluten separation step, comprising washing the starch slurry with an aqueous solution comprising an effective amount of acidic protease activity.

Advantages may be that the higher quality of the final starch product may be obtained and that a shorter steeping time may be used. This is an advantage especially when corn having a high content of horny endosperm is processed.

In the first aspect the invention relates to a process of washing starch the starch slurry obtained from the starch gluten separation step, comprising subjecting the starch slurry to an effective amount of acidic protease activity. A process may comprise the following steps:
a) crop kernels are steeped by soaking in water;
b) kernels are cracked open to release germ;
c) the germ is removed to provide a starch fraction;
d) the starch fraction obtained in step c) is freed from fibers to provide mill starch starch-gluten suspension);
e) the mill starch obtained in step d) is separated into a starch slurry and a protein slurry;
f) washing the starch slurry obtained from step e) with an aqueous solution comprising an effective amount of acidic protease activity;
g) recover starch.

It is to be understood that in an embodiment the acidic protease activity may be residual activity from an earlier come form be

The steeping step may be carried out at a temperature between 40 and 60°C, preferably around 50°C and/or in the presence of 0.01-1%, preferably 0.05-0.3%, especially 0.1% SO₂ and/or NaHSO₃

In a preferred embodiment the starch slurry is further subjected to an effective amount of xylanase activity.

In another aspect the invention relates to a process of washing a starch slurry, comprising subjecting it to an effective amount of acidic protease activity.

The process may comprise the following steps:
a. dry milling of kernels into endosperm grits;
b. separating the milled kernels into a starch slurry and a gluten slurry;
c. washing the starch slurry obtained in step b. with an aqueous solution comprising an effective amount of acidic protease activity;
d. recover starch.

In a preferred embodiment of the invention the dry milled kernels (the grits) obtained in step b. are grinded in wet state (addition of water) until the kernels have an average particles diameter of below 450 micro meters, preferably below 200 micro meters, especially below 100 micro meters and then subjected to step ii).

In a preferred embodiment the starch sluny is further subjected to an effective amount of xylanase activity.

In a further embodiment the starch sluny is also subjected to an effective amount of cellulase and/or arabinofuranosidase.

In a preferred embodiment of the invention the starch slurry is subjected to an effective amount of acidic protease activity to provide pure and high quality starch further comprising in the presence of 0.01-1%, preferably 0.05-0.3%, especially 0.1% SO₂ and/or NaHSO₃.

### Acidic Proteases

Suitable acidic proteases include fungal and bacterial proteases, i.e., proteases characterized by the ability to hydrolyze proteins under acidic conditions below pH 7, preferably below 6, especially below 5.5.

Suitable acid fungal proteases include fungal proteases derived from *Aspergillus, Mucor, Rhizopus, Candida, Coriolus, Endothia, Enthomophtra, Irpex, Penicillium, Sclerotium* and *Torulopsis.* Especially contemplated are proteases derived from *Aspergillus niger (see, e*.*g*., *Koaze et al*., (*1964*), *Agr. Biol. Chem. Japan, 28, 216*), *Aspergillus saitoi (see, e.g., Yoshida, (1954) J. Agr. Chem. Soc*. *Japan, 28*, *66*), *Aspergillus awamori* (Hayashida et al., (1977) Agric. Biol. Chem., 42(5), 927-933, *Aspergillus aculeatus* (WO 95/02044), or *Aspergillus oryzae*; and acidic proteases from *Mucor pusillus* or *Mucor miehei.*

In an embodiment the acidic protease is a protease clomplex from *A. oryzae* sold under the tradename Flavourzyme® (from Novozymes A/S) or an aspartic protease from *Rhizomucor miehei* or Spezyme® FAN or GC 106 from Genencor Int.

In a preferred embodiment the process of the invention is carried out in the presence of the acidic Protease I derived from *A. aculeatus* CBS 101.43 in an effective amount.

The starch slurry is subjected to a composition of the invention as described so that it is subjected to from 4,000-20,000 HUT/100 g DS kernels acidic protease, preferably 5,000-10,000 HUT/100 g DS kernels, especially from 6,000-16,500 HUT/100 g DS kernels.

In a preferred embodiment the acidic protease is an aspartic protease, such as an aspartic protease derived from a strain of *Aspergillus,* in particular *A. aculeatus*, especially *A. aculeatus* CBS 101.43.

Preferred acidic proteases are aspartic proteases, which retain activity in the presence of an inhibitor selected from the group consisting of pepstatin, Pefabloc, PMSF, or EDTA. Protease I derived from *A. aculeatus* CBS 101.43 is such acidic protease.

### Xylanases

In a preferred embodiment of the invention an effective amount of a xylanase activity is also present or added during washing of the starch slurry.

The xylanase activity may be derived from any suitable organism, including fungal and bacterial organisms, such as *Aspergillus, Disporotrichum, Penicillium, Neurospora, Fusarium* and *Trichoderma*.

Examples of suitable xylanases include xylanases derived from *H. insolens* (WO 92/17573; *Aspergillus tubigensis* (WO 92/01793); *A. niger* (Shei et al., 1985, Biotech. and Bioeng. Vol. XXVII, pp. 533-538, and Fournier et al., 1985, Biotech. Bioeng. Vol. XXVII, pp. 539-546; WO 91/19782 and EP 463 706); *A. aculeatus* (WO 94/21785).

In a specific embodiment the xylanase is Xylanase I, II, or III disclosed in WO 94/21785.

Contemplated commercially available xylanase include Shearzyme®, Biofeed wheat® (from Novozymes A/S) and Spezyme® CP (from Genencor Int.).

The xylanase may be added in an amount of 1-100 FXU per 100 g DS kernels, preferably 5-90 FXU per 100 g DS kernels, especially 10-80 FXU per 100 g DS kernels.

### Cellulases

In another preferred embodiment an effective amount of a cellulase activity is also present or added during washing of the starch slurry.

The cellulase may be of microbial origin, such as derivable from a strain of a filamentous fungus (e.g., *Aspergillus, Trichoderma, Humicola, Fusarium).* Specific examples of cellulases include the endo-glucanase (endo-glucanase I) obtainable from *H. insolens* and further defined by the amino acid sequence of fig. 14 in WO 91/17244 and the 43 kD *H. insolens* endoglucanase described in WO 91/17243.

Commercially available cellulase which may be used include Celluclast®, Celluzyme® (available from Novozymes A/S), Spezyme® CP (available from Genencor int.) and Rohament® 7069 W (available from Röhm, Germany).

The cellulase may be added in an amount of 1-1,000 NCU per 100 g DS kernels, preferably 170-900 NCU per 100 g DS kernels, especially 200-800 NCU per 100 g DS kernels.

### Arabinofuranosidases

In an even further embodiment of the process of the invention an effective amount of an arabinofurasidase activity is also present or added during washing of the starch slurry.

Examples of contemplated arabinofuranosidases include *A. niger* alpha-L-arabinofuranosidase A and B disclosed in WO 97/42301; the *Aspergillus* sp. arabinofuranosidase disclosed in EP 871,745; the *Aspergillus niger* K1 alpha-L- arabinofuranosidase disclosed in DD 143925.

### Other enzyme activities

According to the invention an effective amount of one or more of the following activities may also be present or added during washing of the starch slurry: endoglucanase, beta-glucanase, pentosanase, pectinase, arabinanase, xyloglucanase activity, or mixtures thereof.

In an embodiment of the invention the enzyme activities added during the process of the invention is derived from the enzyme product Steepzyme® further enriched with one or more of the following activities: xylanase, cellulase, arabinosidase, endoglucanase, beta-glucanase, pentosanase, pectinase, arabinanase, xyloglucanase and/or acidic protease activity.

It is believed that the acidic protease removes insoluble protein and solubles thereby providing amore pure starch product.

### Composition of the invention

The invention also relates to an enzyme composition. For starch gluten separation the composition may comprise an acidic activity only or a combination of an acidic protease activity and one or more enzyme activities.

In this aspect the invention relates to a composition suitable for washing of a starch slurry comprising one or more of the following enzyme activities: endoglucanase, beta-glucanase, xylanase, cellulase, pentosanase, pectinase, arabinofurasidase, arabinanase, xyloglucanase and/or acidic protease activity.

In a preferred embodiment the composition comprises an acidic protease activity. The composition may further comprise endoglucanase, beta-glucanase, xylanase, cellulase, pentosanase, pectinase, arabinofurasidase, arabinanase, xyloglucanase and/or cellulase activity.

In a preferred embodiment the composition further comprises a xylanase. The composition may also comprise an arabinofurasidase and/or a cellulase activity.

In an embodiment the composition of the invention is the enzyme product Steepzyme® further enriched with xylanase activity and/or cellulase activity and/or arabinofuranosidase activity and/or an acidic protease activity, especially an acidic protease activity.

In an embodiment the composition of the invention comprising more that 3740 HUT/g and/or more that 45 FXU/g and/or more that 1694 NCU/g.

The enzyme composition of the invention may in an embodiment comprise a mono-component Protease I from *A. aculeatus* CBS 101.43. It may further comprise Xylanase II from *A. aculeatus* CBS 101.43 (WO 94/21785).

### Use of a Composition of the Invention

A composition of the invention may be used for washing of a starch slurry.

### MATERIALS & METHODS

### Enzymes:

Steepzyme®: multi activity enzyme complex derived from A. aculeatus 101.43(is available from Novozymes A/S on request)

Shearzyme®: *A. aculeatus* CBS 101.43 xylanase II disclosed in WO 94/21785 (is available from Novozymes A/S)

Flavourzyme®: multi proteolytic activity enzyme complex derived from *A. oryzae* (is available from Novozymes A/S)

Protease I: acidic protease from *Aspergillus aculeatus* CBS 101.43 (disclosed in WO 95/02044).

### METHODS

### Determination of protease HUT activity;

The HUT activity was determined according to the AF92/2 method published by Novozymes A/S, Denmark. 1 HUT is the amount of enzyme which, at 40°C and pH 4.7 over 30 minutes forms a hydrolysate from digesting denatured hemoglobin equivalent in absorbancy at 275 nm to a solution of 1.10 µg/ml tyrosine in 0.006 N HCl which absorbancy is 0.0084. The denatured hemoglobin substrate is digested by the enzyme in a 0.5 M acetate buffer at the given conditions. Undigested hemoglobin is precipitated with trichloroacetic acid and the absorbance at 275 nm is measured of the hydrolysate in the supernatant.

### Determination of xylanase activity (FXU)

The endo-xylanase activity is determined by an assay, in which the xylanase sample is incubated with a remazol-xylan substrate (4-O-methyl-D-glucurono-D-xylan dyed with Remazol Brilliant Blue R, Fluka), pH 6.0. The incubation is performed at 50°C for 30 min. The background of non-degraded dyed substrate is precipitated by ethanol. The remaining blue colour in the supernatant is determined spectrophotometrically at 585 nm and is proportional to the endoxylanase activity. The endoxylanase activity of the sample is determined relatively to an enzyme standard. The assay is further described in the publication AF 293.6/1-GB, available upon request from Novozymes A/S, Denmark.

### Determination of Endo-Glucanase Units (ECU)

The ECU (endocellulose unit) is determined relatively to an enzyme standard.

Endocellulase decomposes carboxylmethylcellulose, CMC. The resulting reduction in viscosity is determined by a CMC-vibration Viscosimeter (e.g. MIVI 3000 available from Sofraser, France). The prepared substrate solution contain 35 g/l CMC (Blanose Aqualon) in 0.1 M phosphate buffer at pH 7.5. The enzyme sample to be analyzed is determined is dissolved in the same buffer. 0.15 ml standard enzyme solution or the unknown enzyme sample is placed in 10 ml test tubes. 5 ml CMC-substrate solution, preheated to 40°C, is added. The joint solution is mixed thoroughly, incubated for 30 minutes and placed in the viscometer. The method is further described in AF302/1-GB available from Novozymes A/S upon request.

### Determination of endo-glucanase activity (EGU)

The fermentation broths are analyzed by vibration viscosimetry on CMC at pH 6.0. More specifically, a substrate solution containing 34.0 g/l CMC (Blanose Aqualon) in 0.1 M phosphate buffer, pH 6.0 is prepared. The enzyme sample to be analyzed is dissolved in the same buffer. 14 ml substrate solution and 0.5 ml enzyme solution are mixed and transferred to a vibration viscosimeter (e.g. MIVI 3000 available from Sofraser, France) thermostated at 40°C. Endoglucanase unit (EGU) is determined as the ratio between the viscosity of the sample and the viscosity of a standard enzyme solution.

### Cellulytic Activity (NCU)

The cellulytic activity is determined with carboxymethyl cellulose (CMC) as substrate.

One Novo Cellulase Unit (NCU) is defined as the amount of enzyme which, under standard conditions (i.e. at pH 4.80; 0.1 M acetate buffer; 10 g/l Hercules CMC type 7 LFD as substrate; an incubation temp. of 40.0°C; an incubation time of 20 min; and an enzyme concentration of approximately 0.041 NCU/ml) forms an amount of reducing carbohydrates equivalent to 1 micro mol glucose per minute. A folder AF 187.2/1 describing this analytical method in more detail is available upon request to Novozymes A/S, Denmark, which folder is hereby included by reference.

### Arabinofuranosidase assay

The synthetic substrate p-nitrophenyl alpha-L-arabinofuranoside (SIGMA) is used as substate. Following cleavage of the enzyme, the p-nitrophenyl molecule is liberated and the development in yellow colour can be measured by visible spectrometty at 405 nm.

Stock solution: 1 mg/ml p-nitrophenyl alpha-L-arabinofuranoside in DMSO.

Substrate solution: 0.2 mg/ml p-nitrophenyl alpha-L-arabinofuranoside diluted in 50 mM Sodium acetate, pH 4.5.

Procedure: 100 microlitre enzyme and 100 microlitre is mixed in a 96-well plate and the development of yellow colour due to the enzymatic reaction is measured from 0 to 15 minutes at 405 nm. The slope of the time dependent OD405 curve is directly proportional to the amount of alpha-arabinofuranosidase.

### Example 1

### Enzymatic cleaning of starch

Starch from wheat or corn may be slightly colored due to attachment of mainly protein, carbohydrate material and xanthophylls for corn.

For some applications an almost complete white starch is wanted. For corn a reduced steeping time (treatment with sulphur dioxide) may result in a slightly yellow starch product due to attachment of protein and xanthophylls. It is the idea to carry out a cleaning of the starch with an effective amount of an acidic protease after the starch-gluten centrifugation but before the hydrocyclones, in the wet milling process. At this stage up to about 500 ppm free sulphur dioxide may be present in the starch fraction to be cleaned, and pH is 4-4.5. Tests have shown that Viscozyme and/or acidic protease can be used on cornstarches. Viscozyme in a dosage of 0.2-0.5 % of starch dry matter have shown a reasonably good effect in our trials with cornstarch even though the level of protease is not that high.

### Results:

The yellowness as measured in Hunter's B-value showed an effect of the enzyme treatment. The starch treated with the acidic protease was as white as the commercial starch sample.

## Claims

1. A process of washing a starch slurry obtained from the starch gluten separation step of a milling process, comprising washing the starch slurry with an aqueous solution comprising acidic protease activity derived from Aspergillus aculeatus, wherein said acidic protease activity is present in an amount of 3,000-6,000 HUT/ 100 g kernels.

2. The process of claim 1, wherein the starch slurry is further subjected to a xylanase in an amount of 1-100 FXU per 100 g DS kernels, preferably 5-90 FXU per 100 g kernels, especially 10 to 80 FXU per 100 g DS kernels.

3. The process of claim 1 or 2, wherein further one or more of the following enzyme activities are added: arabinofuranosidase, cellulase, endoglucanase, beta-glucanase, pentosanase, pectinase, arabinanase, and/or xyloglucanase, or mixtures thereof.

4. A composition comprising a xylanase and an acidic protease activity, wherein the xylanase is present in an amount of more than 45 FXU/g, and wherein the acidic protease activity is present in an amount of more than 3740 HUT/g.

5. The composition of claim 4 further comprising one or more of the following enzyme activities: endoglucanase, beta-glucanase, cellulase, pentosanase, pectinase, arabinofuranosidase, arabinanase and xyloglucanase.

6. Use of a composition of any of claims 4 and 5 for washing a starch slurry.

7. A process of washing a starch slurry obtained from the starch gluten separation step of a milling process, comprising washing the starch slurry with an aqueous solution comprising an effective amount of acidic protease activity.

8. A process comprising the following steps:
a) crop kernels are steeped by soaking in water;
b) kernels are cracked open to release germ;
c) the germ is removed to provide a starch fraction;
d) the starch fraction obtained in step c) is freed from fibers to provide mill starch starch-gluten suspension;
e) the mill starch obtained in step d) is separated into a starch slurry and a protein slurry;
f) washing the starch slurry obtained from step e) with an aqueous solution comprising an effective amount of acidic protease activity;
g) recovering starch.

9. The process of claims 7 or 8, wherein the starch slurry is further subjected to an effective amount of a xylanase activity.

10. A process comprising the steps of:
a. dry milling of kernels into endosperm grits;
b. separating the milled kernels into a starch slurry and a gluten slurry;
c. washing the starch slurry obtained in step b. with an aqueous solution comprising an effective amount of an acidic protease activity;
d. recovering starch.

11. The process of claim 10, wherein the dry milled kernels (the grits) obtained in step a. are grinded in wet state until the kernels have an average particles diameter of below 450 micro meters, preferably below 200 micro meters, especially below 100 micro meters and then subjected to step c..

12. The process of daims 7-11, wherein the starch slurry is further subjected to an effective amount of a xylanase activity.

13. The process of claims 7-12, wherein xylanase is added in an amount of 1-100 FXU per 100 g DS kernels, preferably 5-90 FXU per 100 g kernels, especially 10 to 80 FXU per 100 g DS kernels.

14. The process of claims 7-13, wherein the starch sluny is washed in the presence of an effective amount of a cellulase.

15. The process of claim 7-14, wherein cellulase is added in an amount of 1-1000 NCU per 100 g DS kernels, preferably 170-900 NCU per 100 g DS kernels, especially 200 to 800 NCU per 100 g DS kernels.

16. The process of daims 7-15, wherein the starch slurry is washed in the presence of an effective amount of an arabinofuranosidase.

17. The process of claims 7-16, wherein the acidic protease is added in an amount of 1-10,000 HUT/100 g kernels, preferably 300-8,000 HUT/100g kernels, especially 3,000-6,000 HUT/ 100 g kernels.

18. The process of claim 7-17, wherein further one or more of the following enzyme activities are added: endoglucanase, beta-glucanase, pentosanase, pectinase, arabinanase, and/or xyloglucanase, or mixtures thereof.

19. A process of claims 7-1B, wherein steeping of kernels is carried out at a temperature between 40 and 60°C, preferably around 50°C.

20. The process of claims 7-19, wherein the steeping step is carried out in the presence of 0.01-1%, preferably 0.05-0.3%, especially 0.1% SO₂ and/or NaHSO₃,

21. The process of claim 7-20, wherein the subjection of the milled kernels to an effective amount of an acidic protease activity to provide a starch and a gluten slurry is performed in the presence of 0.01-1%, preferably 0.05-0.3%, especially 0.1% SO₂ and/or NaHSO₃.

22. The process of claims 7-21, wherein the crop kernels are from corn (maize), rice, barley, sorghum bean, or fruit hulls, or wheat.

23. The process of claims 7-22, wherein the xylanase, and/or cellulase, and/or arabinofurasidase and/or acidic protease is derived from the genus *Aspergillus***,** preferably *A. aculeatus*, especially *A. aculeatus* CBS 101.43.

24. The process of claims 7-23, wherein the enzyme activities is derived from Steepzyme® enriched with one or more of the following activities: xylanase, cellulase, arabinosidase, endoglucanase, beta-glucanase, pentosanase, pectinase, arabinanase, xyloglucanase and/or acidic protease activity.

25. The process of claims 7-24, wherein the kernels are subjected to the Steepzyme® enzyme activities enriched to provide a total HUT/100 g DS kernels from 4,000-20,000 HUT/100 g DS kernels acidic protease, preferably 5,000-10,000 HUT/100g, especially from 6,000-16,500 HUT/g DS kernels.

26. The process of claims 7-25 wherein the acidic protease is an aspartic protease, such as an aspartic protease derived from a strain of *Aspergillus,* in particular *A. aculea*tus, especially *A. aculeatus* CBD 101.43.

27. The process of claims 7-26, wherein the aspartic protease retains activity in the presence of an inhibitor selected from the group consisting of pepstatin, Pefabloc, PMSF, and EDTA.

28. The process of claims 7-27, wherein the acidic protease is protease I derived from *A. aculeatus* CBS 101.43.

29. A composition comprising one or more of the following enzyme activities: endoglucanase, beta-glucanase, xylanase, cellulase, pentosanase, pectinase, arabinofurasidase arabinanase, xyloglucanase and/or acidic protease activity.

30. The composition of claim 29 comprising a xylanase and an acidic protease activity.

31. The composition of claim 30, wherein the composition further comprises arabinofurasidase and/or cellulase activity.

32. The composition of claim 29-31, wherein the composition is Steepzyme® enriched with a cellulase and/or a xylanase and/or an arabinofuranosidase and/or an acidic protease.

33. The composition of claim 29-32, comprising more that 3740 HUT/g.

34. The composition of claim 29-33, comprising more that 45 FXU/g.

35. The composition of claim 29-33, comprising more that 1694 NCU/g.

36. Use of a composition of claims 29-35, for washing a starch slurry obtained from a starch gluten separating process.

37. Use of a composition of claims 29-35 for washing a starch slurry.

38. Use of a composition of claims 29-35 in a process of claims 7-28.

39. Use of claims 36-38, for washing the starch slurry obtained from a corn or sorghum wet or dry milling process.
